**Europäisches Patentamt**

**European Patent Office**

**Office européen des brevets**

(19)

(11) Veröffentlichungsnummer : **0 068 450 B2**

(12) **NEUE EUROPÄISCHE PATENTSCHRIFT**

(45) Veröffentlichungstag der neuen Patentschrift :
**09.06.93 Patentblatt 93/23**

(21) Anmeldenummer : **82105579.5**

(22) Anmeldetag : **24.06.82**

(51) Int. Cl.⁵ : **A61K 9/20,** A61K 9/48,
A61K 47/00

(54) **Arzneiformen zur oralen Verabreichung.**

Die Akte enthält technische Angaben, die nach dem Eingang der Anmeldung eingereicht wurden und die nicht in dieser Patentschrift enthalten sind.

(30) Priorität : **25.06.81 DE 3124983**

(43) Veröffentlichungstag der Anmeldung :
**05.01.83 Patentblatt 83/01**

(45) Bekanntmachung des Hinweises auf die Patenterteilung :
**10.01.90 Patentblatt 90/02**

(45) Bekanntmachung des Hinweises auf die Entscheidung über den Einspruch :
**09.06.93 Patentblatt 93/23**

(84) Benannte Vertragsstaaten :
**AT BE CH DE FR GB IT LI LU NL**

(56) Entgegenhaltungen :
**EP-A- 0 001 822**
**DE-A- 1 952 304**
**DE-A- 2 710 307**
**DE-A- 2 736 794**
**DE-A- 2 815 578**
**DE-B- 1 175 826**
**DE-B- 1 195 012**
**DE-B- 2 400 819**
**GB-A- 1 113 860**
**US-A- 3 374 146**
**US-A- 4 132 753**
**Deutsches Arzneibuch 7. Ausgabe 1968, seite 406**
**H. Sucker et al. "Pharmazeutische Technologie", 1978, S. 349-356**
**Hagers Handbuch der Pharmazeutischen Praxis, 1971, S. 689, 690, 729**
**F. Müller "Galenische Möglichkeiten und Probleme bei der Protrahierung von Arzneimittelwirkungen" Arzneimittelforschung-Drug Research, 1976, S. 115-121**
**W.A. Ritschel "Entwicklung einer peroralen Proxyphyllin-Retardform" 3. Mitteilung, Pharmazeutische Zeitung Nr. 47, 1968, S. 1881-1885**
**W.A. Ritschel "Angewandte Biopharmazie", 1973, S. 310-320**

(56) Entgegenhaltungen :
**P.Fiedler "Lexikon der Hilfsstoffe für Pharmazie, Kosmetik und Pharmazeutika und angrenzende Gebiete", 1981, S. 748-750**
**R. Voigt "Lehrbuch der pharmazeutischen Technologie", 1982, 4. Auflage, Seiten 484-486**

(73) Patentinhaber : **Dr. Rentschler Arzneimittel GmbH & Co.**
**Postfach 320**
**W-7958 Laupheim 1 (DE)**

(72) Erfinder : **Lahr, Wolfgang**
**Hopfenweg 18**
**W-7958 Lauhpheim (DE)**
Erfinder : **Muskulus, Frank**
**Gartenweg 1**
**W-7959 Mietingen-Baltringen (DE)**

(74) Vertreter : **Sandmair, Kurt, Dr. et al**
**Patentanwälte Schwabe, Sandmair, Marx**
**Stuntzstrasse 16**
**W-8000 München 80 (DE)**

EP 0 068 450 B2

## Beschreibung

Die Erfindung betrifft ein Verfahren zur Herstellung fester pharmazeutischer Präparate mit verzögerter Wirkstoff-Freigabe, bei dem man den oder die Wirkstoffe in eine nicht-wasserlösliche, geschmolzene Komponente zusammen mit üblichen Trägerstoffen einbringt und die Schmelze zu Körpern vergießt, die die einnahmefähigen Darreichungsformen darstellen.

Feste, zur oralen Verabreichung vorgesehene Arzneiformen werden vornehmlich in zwei Arten aufgeteilt. Entsprechend dem Therapiewunsch soll entweder der gesamte Arzneiwirkstoff innerhalb kurzer Zeit aus der Darreichungsform zur Resorption zur Verfügung gestellt, oder im anderen Fall langsam, über Stunden verzögert, jedoch gleichmäßig retardiert im Verdauungstrakt aus der Darreichungsform freigesetzt werden.

Beide Darreichungsformen sind seit langem bekannt, weisen jedoch den empfindlichen Nachteil des großen technischen und zeitlichen Aufwandes bis zur Erreichung der fertigen Arzneiform auf.

Zur Herstellung einer festen Darreichungsform mit sofortiger Wirkstofffreisetzung sind in der Regel folgende Arbeitsschritte erforderlich:

Zur Erzielung einer zeitlich gesteuerten Freigabe der Wirkstoffe unter verschiedenen Milieubedingungen sind über die oben beschriebenen Arbeitsschritte hinaus weitere Fertigungsstufen erforderlich. So kann beispielsweise ein Retardeffekt erzielt werden, indem die Lösungsgeschwindigkeit eines Wirkstoffes durch die Verwendung von Makrokristallen verringert wird, durch Bindung an makromolekulare Hilfsstoffe, durch Umhüllung mit Diffusionsmembranen, durch gemeinsames Verpressen von Wirkstoffen mit schwer- oder nichtlöslichen Hilfsstoffen oder die Kombination derartiger Verfahren.

Es wurden ferner in der DE-AS 2 126 810, DE-PS 2 127 683, DE-OS 2 904 310, DE-OS 2 439 538, DE-OS 1 952 304, US-PS 2 887 438, AT-PS 130 794 und der Diss. 5768 der ETH Zürich Vorschläge gemacht, Fette, Wachse oder fettähnliche, wasserunlösliche Massen zur Einbettung von Wirksubstanzen zu verwenden, die den darin enthaltenen Wirkstoff retardiert freisetzen sollen.

Neben dem technologischen Aufwand weisen diese Verfahren den empfindlichen Nachteil auf, daß bei der Herstellung derartiger Formlinge, Partikel verschiedener Geometrie und damit unterschiedlicher Freisetzungscharakteristiken anfallen. Die erforderliche Klassierung zur Erzielung reproduzierbarer Ergebnisse bringt in der Regel erhebliche Substanzverluste mit sich, so daß diese Verfahren als unwirtschaftlich angesehen werden müssen.

Neuere Vorschläge, wie sie in der DE-OS 2 838 387 beschrieben sind, gehen von thixotropen Flüssigkeiten aus, die in Kapseln abgefüllt werden, was den Nachteil hat, daß man in der Anwendungsbreite von den rheologischen Eigenschaften der Zusammensetzung abhängig ist. Nichtthixotrope Mischungen finden demnach keine Verwendung.

Die Europäische Patentanmeldung EP-A-1822 beschreibt die Verwendung von thixotropen, wasserlöslichen Flüssigkeiten oder Schmelzmassen zur Abfüllung in Hartgelatine-Steckkapseln auf der Basis von Polyethylenglykolen. Die offensichtlichen Nachteile dieser Zusammensetzungen sind, daß die rheologischen Eigenschaften die Anwendungsbreite begrenzen, andererseits wegen der Wasserlöslichkeit der Matrix keine gezielte Steuerung der Wirkstofffreigabe möglich ist.

Weiterhin ist die DE-A-2 710 307 zu nennen, die ein kombiniertes Dosierungs- und Konfektionierungsverfahren beschreibt, bei dem man eine aktive pharmazeutische Komponente mit einer bei Raumtemperatur festen, bei Erwärmen flüssigen, Trägersubstanz vermischt, und das Gemisch dann auf ein fortlaufendes Band aus Kunststoff, Metall, Cellulose oder ein Laminat aus diesen Stoffen aufbringt, wobei das Band Vertiefungen, die der gewünschten Dosiseinheit entsprechen, aufweist. Das Band dient gleichzeitig der Verpackung. Als Trägersubstanz werden neben vielen anderen auch Cetyl- und Stearylalkohol, die bis zu einer Konzentration von bis nahezu 100% eingesetzt werden können, genannt.

In der DE-B 11 75 826 wird ein Verfahren zur Herstellung von Retard-Arzneimitteln offenbart, gemäß dem sogenannte Multiple dose-Formen dadurch hergestellt werden, daß man ein Gemisch schmilzt, das neben Polyvinylpyrrolidon einen wachsartigen, praktisch wasserunlöslichen, oberhalb 45 °C schmelzenden Träger enthält, der beispielsweise ein Fettalkohol sein kam. Die Arzneipräparate gemäß dieser Druckschrift enthalten im wesentlichen leicht lösliche Wirkstoffe. Polyvinylpyrrolidon spielt in den hergestellten Formulierungen eine entscheidende Rolle im Hinblick auf den Träger, der die retardierte Freisetzung des Arzneimittels garantiert. Die Arzneipräparate gemäß der Druckschrift werden in einem aufwendigen, zahlreiche Verfahrensschritte umfassenden Verfahren zu kleinen Kügelchen verformt, welche in eine spezielle Dosierungseinheit eingekapselt oder einverleibt werden. Das in der Druckschrift beschriebene Verfahren wurde jedoch in der Praxis regelmäßig als zu aufwendig empfunden, insbesondere dann, wenn es um die Herstellung von sogenannten Unit dose-Verabreichungsformen ging.

Es wurde nun ein Verfahren gefunden, bei dem es unter Anwendung bekannter Technologien in drei Herstellungsschritten, Schmelzen→Mischen→Abfüllen bzw. Formen, möglich ist, feste Darreichungsformen mit verzögerter Wirkstoff-Freigabe herzustellen, indem man einen wasserunlöslichen Fettalkohol oder Fettalkoholgemische mit Kettenlängen von 12 bis 24 C-Atomen in einem Anteil von 18 bis 61 Gew.- %, bezogen auf das Gesamtgewicht, als Matrixsubstanz verwendet. Der Fettalkohol oder die Fettalkoholgemische können allein oder zusammen mit wasserlöslichen und/oder sedimentationsverhindernden Hilfsstoffen und/oder lösungsfördernden Hilfsstoffen eingesetzt werden.

Es wurde weiter gefunden, daß durch quantitative Änderungen der Hilfsstoffmengen niedrig und hochdosierte Wirkstoffe sofort oder retardiert freigesetzt werden können.

Als besonders gut geeignet wurden Cetylalkohol, Stearylalkohol, Cetylstearylalkohol und/oder deren Gemische gefunden. Als wasserlösliche Komponente können beispielsweise mehrwertige Alkohole wie Polyethylenglykole mit mittleren Molekulargewichten zwischen 1000 und 20 000 und deren Gemische und/oder Polymerisate aus Vinylpyrrolidon und/oder Mischpolymerisate aus Vinylpyrrolidon und Vinylacetat verwendet werden.

Je nach gewünschter Freisetzungscharakteristik ist die Zugabe von anderen Lösungsvermittlern zweckmäßig, beispielsweise von nichtionogenen Tensiden wie Polyethylen-Fettalkoholethern oder Polyoxyethylen-Sorbitan-Fettsäureestern. Zur Viskositätserhöhung und Verhinderung von Sedimentation während des Herstellprozesses eignet sich beispielsweise kolloidale Kieselsäure.

Im erfindungsgemäßen Verfahren sind alle Wirksubstanzen synthetischen oder natürlichen Ursprungs zur Verarbeitung geeignet, sofern es sinnvoll ist, sie aufgrund ihrer chemisch-physikalischen und pharmakologischen Eigenschaften peroral zu verabreichen. Die Verarbeitung der vorbeschriebenen Komponenten erfolgt im wesentlichen aus der Herstellung einer Schmelze der wasserunlöslichen Fettalkohole, je nach Notwendigkeit der mechanischen Einarbeitung der anderen bekannten Hilfsstoffkomponenten und der Lösung bzw. Suspension der Wirkstoffe in der Schmelze.

Die Abfüllung der beschriebenen Schmelzen kann mit bekannten Gerätschaften, beispielsweise Dosierpumpen, in Kapseln erfolgen.

Die Formung der beschriebenen Schmelzen kann beispielsweise derart erfolgen, daß man gleiche Mengen Schmelze auf einer gekühlten Unterlage abtropft oder in Form vergießt. Nach dem Erstarren erhält man in allen Fällen die fertige Darreichungsform. Die Größe der Kapseln bzw. der Formlinge richtet sich nach der zu verabreichenden Menge Wirkstoff, bzw. der gewünschten Zeit, in welcher der oder die Wirkstoffe zur Resorption freigesetzt werden sollen.

Solchermaßen hergestellte Arzneimittel vereinigen in vorzüglicher Weise die Forderungen nach niedrigen Produktionskosten, einfacher Herstellungstechnologie, guter Reproduzierbarkeit, exakter Dosierung der Wirkstoffe in der einzelnen Darreichungsform, wahlweise zeitabhängiger, gleichmäßiger Freisetzung der Wirksubstanzen in therapeutisch erwünschten Dosen aus der Darreichungsform unter den verschiedenen im Verdauungstrakt auftretenden Milieubedingungen, vollständiger Freisetzung, Schutz der Wirkstoffe gegen äußere Einflüsse wie Licht, Feuchtigkeit und Sauerstoff.

Nachfolgend wird beispielhaft aufgezeigt, wie variabel das Verfahren ist, in dem sowohl hoch als auch niedrig dosierte Wirkstoffe in einer lediglich quantitativ veränderten Matrix verarbeitet werden.

Beispiel 1

| | |
|---|---|
| Stearylalkohol | 30,0 g |
| Polyvinylpyrrolidon | 6,0 g |
| Kolloidale Kieselsäure | 2,0 g |
| Polyoxyethylen-Sorbitan- | |
| Fettsäureester (Tween ® 80) | 16,0 g |
| Codeinphosphat | 6,0 g |

In die Schmelze des Stearylalkohols, 10°C oberhalb seines Schmelzpunktes, werden Polyvinylpyrrolidon, kolloidale Kieselsäure und Tween ® durch Rühren zugemischt und Codeinphosphat darin suspendiert.

Die flüssige Mischung wird in handelsüblichen Gelatine-Steckkapseln Gr. 3 zu je 300 mg pro Kapsel abgefüllt: Wirkstoffgehalt pro Kapsel = 30 mg.

Beispiel 2

| | |
|---|---|
| Stearylalkohol | 26,0 g |
| Polyvinylpyrrolidon | 4,0 g |
| Kolloidale Kieselsäure | 2,0 g |
| Tween ® 80 | 16,0 g |
| Dextromethorphan x HBr | 12,0 g |

Die Verarbeitung erfolgt wie in Beispiel 1. Die flüssige Mischung wird in handelsüblichen Gelatinesteckkapseln Gr. 3 zu je 300 mg pro Kapsel abgefüllt. Wirkstoffgehalt pro Kapsel = 60 mg.

Beispiel 3

| | |
|---|---|
| Stearylalkohol | 28,5 g |
| Kolloidale Kieselsäure | 1,5 g |
| Kaliumchlorid | 30,0 g |

Die Verarbeitung erfolgt wie in Beispiel 1. Die flüssige Mischung wird in handelsüblichen Gelatinesteckkapseln Gr. 00 zu je 580 mg pro Kapsel abgefüllt. Wirkstoffgehalt pro Kapsel = 290 mg.

Beispiel 4

| | |
|---|---|
| Stearylalkohol | 36,40 g |
| Polyvinylpyrrolidon | 13,00 g |
| Kolloidale-Kieselsäure | 2,70 g |
| Tween ® 80 | 7,00 g |
| Dihydroergotaminmethansulfonat | 0,90 g |

Die Verarbeitung erfolgt wie in Beispiel 1. Die flüssige Mischung wird in handelsüblichen Gelatinesteckkapseln Gr. 3 zu je 300 mg pro Kapsel abgefüllt. Wirkstoffgehalt pro Kapsel = 4,5 mg.

Beispiel 5

| | |
|---|---|
| Stearylalkohol | 10,8 g |
| Polyethylenglykol (mittl. Mol.-Gew. 2000) | 18,0 g |
| Kolloidale Kieselsäure | 1,2 g |
| Kaliumchlorid | 30,0 g |

Die Verarbeitung erfolgt wie in Beispiel 1. Die flüssige Mischung wird in handelsüblichen Gelatinesteckkapseln Gr. 00 zu je 580 mg pro Kapsel abgefüllt. Wirkstoffgehalt pro Kapsel = 290 mg.

In vitro Wirkstoff-Freisetzung bei verschiedenen pH-Werten und einer konstanten Temperatur von 37°C ± 1 °C in Abhängigkeit von der Zeit.

| Zeit | pH | Bsp. 1 | Bsp. 2 | Bsp. 3 | Bsp. 4 | Bsp. 5 |
|------|----|--------|--------|--------|--------|--------|
| 1 h | 1.5 | 24.1% | 37.0% | 20.0% | 19.5% | 83.7% |
| 2 h | 4.5 | 17.0% | 21.9% | 14.1% | 28.0% | – |
| 3 h | 6.9 | 23.9% | 14.4% | 11.6% | 20.2% | – |
| 5 h | 6.9 | 17.1% | 17.1% | 16.2% | 18.5% | – |
| 7 h | 7.2 | 16.2% | 11.7% | 11.6% | 11.6% | – |
| Restbest. | | 3.5% | – | 26.5% | 2.2% | 14.7% |

## Beispiel 6

| | |
|---|---|
| Stearylalkohol | 72,80 g |
| Polyvinylpyrrolidon | 26,00 g |
| Kolloidale Kieselsäure | 5,40 g |
| Tween ® 80 | 14,00 g |
| Dihydroergotaminmethansulfonat | 4,20 g |

Die Verarbeitung erfolgt wie in Beispiel 1. Die flüssige Mischung wird auf eine gekühlte, glatte und porenfreie Unterlage mittels Dosierpumpe zu gleichen Volumina abgetropft. Man erhält pastillenartige Formlinge zu durchschnittlich 56,6 mg Gewicht und einem Durchmesser von 6,1 mm mit einem Gehalt an Wirkstoff von 2,0 mg/Formling.

### Freisetzung

| | |
|---|---|
| 1 Stunde | 24,6% |
| 2 Stunden | 19,5% |
| 3 Stunden | 18,2% |
| 5 Stunden | 23,5% |
| 7 Stunden | 11,3% |
| Restbestimmung | - |

## Patentansprüche

1. Verfahren zur Herstellung fester Darreichungsformen mit verzögerter Wirkstoff-Freigabe, bei dem man
a) den bzw. die Wirkstoffe(e) in eine wasserunlösliche, geschmolzene Komponente zusammen mit üblichen Trägerstoffen einbringt, und
b) die Schmelze zu Körpern vergießt, die die einnahmefertigen Darreichungsformen darstellen, dadurch gekennzeichnet, daß die wasserunlösliche Komponente ein Fettalkohol oder Fettalkoholgemische mit Kettenlängen von 12 bis 24 C-Atomen ist und der Anteil der wasserunlöslichen Komponente, bezogen auf das Gesamtgewicht, 18 bis 61 Gew.% beträgt.

2. Verfahren nach Anspruch 1, dadurch gekennzeichnet, daß als Fettalkohol Cetyalkohol, Stearylalkohol und/oder Cetylstearylalkohol verwendet wird.

3. Verfahren nach Anspruch 1 und 2, dadurch gekennzeichnet, daß zur geschmolzenen Komponente zusätzlich ein oder mehrere wasserlösliche und/oder lösungsvermittelnde und/oder sedimentationsverhindernde Zuschlagsstoffe zugegeben werden.

4. Verfahren nach Anspruch 3, dadurch gekennzeichnet, daß als wasserlösliche Komponente Polyethylenglykole mit einem mittleren Molekulargewicht von 1000 bis 20 000 verwendet werden.

5. Verfahren nach Anspruch 3, dadurch gekennzeichnet, daß als wasserlösliche und lösungsvermittelnde Komponente Polymerisate aus Vinylpyrrolidon und/oder Mischpolymerisate aus Vinylpyrrolidon und Vinylacetat verwendet werden.

6. Verfahren nach Anspruch 3, dadurch gekennzeichnet, daß als lösungsvermittelnde Komponente nichtionogene Tenside verwendet werden.

5

**7.** Verfahren nach Anspruch 3, dadurch gekennzeichnet, daß als lösungsvermittelnde Komponente Polyethylen-Fettalkoholether verwendet werden.

**8.** Verfahren nach Anspruch 3, dadurch gekennzeichnet, daß als lösungsvermittelnde Komponente Polyoxyethylen-Sorbitan-Fettsäureester verwendet werden.

**9.** Verfahren nach Anspruch 3, dadurch gekennzeichnet, daß als sedimentationsverhindernde Komponente kolloidale Kieselsäure verwendet wird.

**10.** Darreichungsform zur oralen Verabreichung mit verzögerter Wirkstoffreigabe, dadurch gekennzeichnet, daß sie nach einem Verfahren gemäß einem der Ansprüche 1 bis 9 hergestellt wurde.

## Claims

**1.** Method for the production of solid dosage forms with delayed release of the active ingredient, in which
a) the active ingredient(s) is/are incorporated together with conventional carrier substances into a molten, water-insoluble component, and
b) the melt is cast into bodies which constitute dosage forms which are ready for use, characterized in that the water-insoluble component is a fatty alcohol or fatty alcohol mixtures with chain lengths of from 12 to 24 C-atoms and the proportion of the component which is insoluble in water, in relation to the total weight, is 18 to 61% by weight.

**2.** Method according to Claim 1, characterized in that cetyl alcohol, stearyl alcohol and/or cetylstearyl alcohol is used as fatty alcohol.

**3.** Method according to Claim 1 and 2, characterized in that additionally one or more water-soluble and/or solubilizing and/or sedimentation-preventing additives are added to the molten component.

**4.** Method according to Claim 3, characterized in that polyethylene glycols with an average molecular weight of from 1 000 to 20 000 are used as water-soluble component.

**5.** Method according to Claim 3, characterized in that polymer of vinyl pyrrolidon and/or mixed polymer of vinyl pyrrolidon and vinyl acetate are used as water-soluble and solubilizing component.

**6.** Method according to Claim 3, characterized in that non-ionic surfactants are used as solubilizing component.

**7.** Method according to Claim 3, characterized in that polyethylene fatty alcohol ethers are used as component.

**8.** Method according to Claim 3, characterized in that polyoxyethylene sorbitan fatty acid esters are used as component.

**9.** Method according to Claim 3, characterized in that colloidal silicic acid is used as sedimentation-preventing component.

**10.** Dosage form for oral administration with delayed release of effective substance, characterized in that it was produced by a method according to one of Claims 1 to 9.

## Revendications

**1.** Procédé de préparation de formes solides d'administration avec libération retardée de la substance active, procédé dans lequel :
a) on introduit la ou les substances actives dans un composant fondu insoluble dans l'eau, conjointement avec des substances supports habituelles, et
b) on coule la masse fondue en corps qui constituent les formes d'administration prêtes à être prises, **caractérisé en ce que** le composant insoluble dans l'eau est un alcool gras ou des mélanges d'alcools gras ayant des longueurs de chaînes de 12 à 24 atomes de carbone tandis que, rapportée au poids total, la quantité du composant insoluble dans l'eau est de 18 à 61% en poids.

2. Procédé selon la revendication 1, **caractérisé en ce que**, comme alcool gras, on utilise l'alcool cétylique, l'alcool stéarylique et/ou l'alcool cétylstéarylique.

3. Procédé selon les revendications 1 et 2, **caractérisé en ce que**, au composant fondu, on ajoute, en outre, un ou plusieurs additifs hydrosolubles et/ou unisseurs et/ou empêchant la sédimentation.

4. Procédé selon la revendication 3, **caractérisé en ce que**, comme composant hydrosoluble, on utilise des polyéthylène-glycols d'un poids moléculaire moyen de 1.000 à 20.000.

5. Procédé selon la revendication 3, **caractérisé en ce que**, comme composants hydrosolubles et unisseurs, on utilise des polymères de vinylpyrrolidone et/ou des copolymères de vinylpyrrolidone et d'acétate de vinyle.

6. Procédé selon la revendication 3, **caractérisé en ce que**, comme composants unisseurs, on utilise des agents tensio-actifs non ionogènes.

7. Procédé selon la revendication 3, **caractérisé en ce que**, comme composants unisseurs, on utilise des éthers d'alcools gras de polyéthylène.

8. Procédé selon la revendication 3, **caractérisé en ce que**, comme composants unisseurs, on utilise des esters d'acides gras de polyoxyéthylène-sorbitanne.

9. Procédé selon la revendication 3, **caractérisé en ce que**, comme composant empêchant la sédimentation, on utilise l'acide silicique colloïdal.

10. Forme de préparation pour l'administration par voie orale avec libération retardée de la substance active, **caractérisée en ce qu'**on la prépare par un procédé selon une des revendications 1 à 9.